(19) Europäisches Patentamt
European Patent Office
Office européen des brevets

(11) **EP 1 711 072 B1**

(12) **EUROPÄISCHE PATENTSCHRIFT**

(45) Veröffentlichungstag und Bekanntmachung des Hinweises auf die Patenterteilung:
**05.10.2011 Patentblatt 2011/40**

(21) Anmeldenummer: **05707809.9**

(22) Anmeldetag: **20.01.2005**

(51) Int Cl.:
*A23L 1/304* *(2006.01)*     *A61K 33/06* *(2006.01)*
*C12P 7/56* *(2006.01)*

(86) Internationale Anmeldenummer:
**PCT/EP2005/050240**

(87) Internationale Veröffentlichungsnummer:
**WO 2005/072539 (11.08.2005 Gazette 2005/32)**

(54) **VERFAHREN ZUR HERSTELLUNG EINER CALCIUM-LAKTAT-MALAT VERBINDUNG UND CALCIUM-LAKTAT-MALAT VERBINDUNG**

METHOD FOR THE PRODUCTION OF A CALCIUM LACTATE MALATE COMPOUND AND CALCIUM LACTATE MALATE COMPOUND

PROCEDE POUR PRODUIRE UN COMPOSE DE LACTATE-MALATE DE CALCIUM ET COMPOSE DE LACTATE-MALATE DE CALCIUM OBTENU SELON LEDIT PROCEDE

(84) Benannte Vertragsstaaten:
**DE FR GB**

(30) Priorität: **28.01.2004 DE 102004004439**
**18.10.2004 DE 102004050703**

(43) Veröffentlichungstag der Anmeldung:
**18.10.2006 Patentblatt 2006/42**

(73) Patentinhaber: **Dr. Paul Lohmann GmbH KG**
**31860 Emmerthal (DE)**

(72) Erfinder:
• **BERLEKAMP, Uwe**
**31860 Emmerthal (DE)**
• **GÜNTHER, Uwe**
**31787 Hameln (DE)**
• **STÄBE, Dietmar**
**31860 Emmerthal (DE)**

(74) Vertreter: **Hannke, Christian**
**Hannke Bittner & Partner**
**Patent- und Rechtsanwälte**
**Ägidienplatz 7**
**93047 Regensburg (DE)**

(56) Entgegenhaltungen:
**WO-A-00/18258     WO-A-92/21355**
**US-A- 4 737 375**

• **DATABASE WPI Section Ch, Week 200453 Derwent Publications Ltd., London, GB; Class B05, AN 1997-314183 XP002324322 -& JP 03 552075 B2 (ORIENTAL YEAST CO LTD) 11. August 2004 (2004-08-11)**
• **DATABASE WPI Section Ch, Week 197909 Derwent Publications Ltd., London, GB; Class D13, AN 1979-16910B XP002324323 & JP 54 008767 A (KAJI H) 23. Januar 1979 (1979-01-23)**
• **DATABASE WPI Section Ch, Week 199932 Derwent Publications Ltd., London, GB; Class B04, AN 1999-371698 XP002324324 & CN 1 212 853 A (JIANG C) 7. April 1999 (1999-04-07)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung bezieht sich auf ein Herstellungsverfahren für eine sehr gut löslichen Calcium-Laktat-Malat Verbindung, damit supplementierte Lebensmittel, die Verwendung für die Herstellung von Lebensmitteln sowie die Verwendung für die Herstellung pharmazeutischer Zubereitungen, insbesondere für die Behandlung von Osteoporose.

Calcium ist ein essentieller Mineralstoff in der Ernährung, der dem Körper täglich in ausreichender Menge zugeführt werden muss. Durch einseitige Ernährung, aber auch durch besondere körperliche Beanspruchung, wie bei Sport, Krankheit u. ä., kann es zu einem Mangel dieses Mineralstoffes kommen, weshalb Lebensmittel, insbesondere Getränke, häufig mit Calcium supplementiert werden.

Calcium ist für den Aufbau der Knochen, für die Blutgerinnung und die Funktion der Muskelkontraktion essentiell. Calciummangel ist ein entscheidender Faktor für die Entstehung von Osteoporose. Die empfohlene täglich Aufnahme von Calcium für einen Menschen liegt bei ca. 800 mg/Tag (RDA = recommended daily allowance gemäß Richtlinie 90/496/EWG).

Hauptquelle für Calcium sind Milch und Milchprodukte, in großem Abstand gefolgt von Obst und Gemüse, Getreideprodukten, Fleisch und Eiern. Daher ist insbesondere bei einer Ernährung mit geringem Anteil an Milch und Milchprodukten, wie sie bei vielen Erwachsenen auf Grund von beispielsweise einer Milchzucker-Unverträglichkeit vorliegt, eine Supplementierung mit Lebensmitteln, die mit Calcium angereichert sind, erwünscht.

Um Mangelzustände bei einseitigen Ernährungsgewohnheiten auszugleichen, werden daher Calciumderivate zur Anreicherung von Lebensmitteln eingesetzt.

Viele der kommerziell erhältlichen Calciumderivate haben den entscheidenden Nachteil, dass sie nur sehr langsam bzw. schwer löslich sind und der enthaltene Calciumanteil nicht nur unvollständig in Lösung geht, sondern häufig gerade bei der Verwendung des Mineralstoffes Calcium, dessen Tagesbedarf der höchste unter den Mineralstoffen ist, nicht in ausreichender Menge für eine Supplementierung gelöst werden kann.

Von Calciumcarbonat, Calciumcitrat und Calciumgluconat ist bekannt, dass sie für die Anreicherung von flüssigen Lebensmitteln mit Calcium ungeeignet sind, da ihre Löslichkeit unzureichend ist. Insbesondere ist auch von Calciummalat bekannt, dass es Calcium nur in sehr geringen Mengen in Lösung bringen kann. Auch Calciumlaktat löst das Calcium nicht in einer für eine hohe Supplementierung ausreichenden Weise und besitzt eine unzureichende Lösegeschwindigkeit.

Insbesondere ist von Calciummalat bekannt, dass es Calcium nur in sehr geringen Mengen in Lösung bringen kann. Auch Calciumlaktat löst das Calcium nicht in einer für eine hohe Supplementierung ausreichenden Weise und besitzt eine nur unzureichende Lösegeschwindigkeit. Zudem ist der Calciumgehalt des Calciumlactats mit ca. 13,5 % bezogen auf die Einwaage sehr gering. Ein Nachteil, den auch das Calciumlactatgluconat mit nur 10,5 % Calciumgehalt, bezogen auf die Einwaage, hat.

Ein entscheidender Nachteil der sehr gut löslichen Verbindung Calciumlactatgluconat ist, dass es bei der für die Lebensmittelherstellung häufig nötigten Erwärmung bereits ab 140 °C zu unschönen Verfärbungen kommt.

Eine Reihe anderer gut löslicher Calciumderivate, wie beispielsweise Calciumacetat und Calciumchlorid haben einen unangenehmen Geschmack, wodurch eine Verwendung bei der Lebensmittelherstellung praktisch ausgeschlossen ist.

Vereinzelt gibt es Hinweise darauf, das Calcium in flüssigen Lebensmitteln in Kombination mit Fruchtsäuren eingesetzt wird, wie beispielsweise in den Patentschriften CN 120587 oder EP0301653. Aus der Patentschrift JP9121811 A (13.05.1997) sind leicht absorbierbare Calcium-Laktat-Malat Verbindungen bekannt. Eine konkrete Beschreibung einzelner isolierten Verbindungen, die ein zielgerichtetes Herstellungsverfahren, bei dem alle Ausgangsstoffe vollständig in das Endprodukt umgesetzt werden, fehlt jedoch.

Der vorliegenden Erfindung liegt daher die Aufgabe zugrunde, ein Verfahren zu entwickeln, das die Herstellung eines Calcium-Derivates durch die vollständige Umsetzung aus den Ausgangsverbindungen ermöglicht, dabei eine Verbindung entstehen lässt, die für die Supplementierung von Lebensmitteln mit Calcium geeignet ist, einen hohen Calciumanteil in Lösung bringt, eine hohe Lösegeschwindigkeit besitzt, keine negative Geschmackskomponente in das Lebensmittel einträgt, Calcium in einem hohen Anteil enthält und zudem auch über 140 °C thermostabil ist. Des Weiteren ist es die Aufgabe der Erfindung ein Lebensmittel mit hohem Calciumgehalt in Bezug auf die empfohlene Tagesdosierung von Calcium bereitzustellen, dessen Geschmack durch den hohen Calciumgehalt nicht negativ beeinträchtigt wird, ein Herstellungsverfahren für ein derartiges Lebensmittel zu finden und ein entsprechendes Calcium-Derivat für die Verwendung in pharmazeutischen Zubereitungen zugänglich zu machen.

Diese Aufgabe wird verfahrensseitig durch die Merkmale der Patentansprüche 1, 4 und 15, stoffseitig durch die Merkmale der Patentansprüche 8 und 12 und verwendungsseitig durch die Merkmale der Patentansprüche 19 und 20 gelöst.

Ein wesentlicher Punkt der Erfindung liegt darin, dass bei einem Verfahren zur Herstellung eines Calcium-Derivats folgende Schritte durchgeführt werden:

- Lösen vorbestimmter Anteile an Milchsäure und Äpfelsäure in Wasser zur Bildung einer Lösung;
- Zugeben vorbestimmter Anteile an Calciumhydroxid, Calciumcarbonat und/oder Calciumoxid zu der Lösung und
- Entfernen des Wasseranteils der Lösung zur vollständigen Umsetzung der vorbestimmten Anteile zu einer Calcium-Laktat-Malat Verbindung mit der folgenden Verhältnisformel: $Ca^{2+} (C_3O_3H_5)^-_x (C_4O_5H_4)^{2-}_y$,

wobei x und y die molaren Verhältnisse von Laktat bzw. Malat bezogen auf 1 Mol Calcium in dieser Calcium-Laktat-Malat Verbindung angeben, x+ 2 y = 2 gilt,
der molare Anteil des Laktats in einem Bereich von x = 1,7 - 1,98 liegt und der molare Anteil des Malats in einem Bereich von y = 0,15 - 0,01 liegt.

Alternativ weist das erfindungsgemäße Herstellungsverfahren folgende Schritte auf:

- Lösen vorbestimmter Anteile von Calicumlaktat und Calciummalat in Wasser zu einer Lösung und
- Entfernen des Wasseranteils der Lösung zur vollständigen Umsetzung der vorbestimmten Anteile zu einer Calcium-Laktat-Malat Verbindung mit der folgenden Verhältnisformel: $Ca^{2+} (C_3O_3H_5)^-_x (C_4O_5H_4)^{2-}_y$,

wobei x und y die molaren Verhältnisse von Laktat bzw. Malat bezogen auf 1 Mol Calcium in dieser Calcium-Laktat-Malat Verbindung angeben,

$$x+ 2\, y = 2 \text{ gilt,}$$

der molare Anteil des Laktats in einem Bereich von x = 1,7 - 1,98 liegt und der molare Anteil des Malats in einem Bereich von y = 0,15 - 0,01 liegt.

Hierdurch ist vorteilhaft eine Calcium-Laktat-Malat Verbindung erhältlich, die durch vollständige Umsetzung der eingesetzten Ausgangsstoffe, ohne dass dabei Anteile der Ausgangsstoffe zurückbleiben, herstellbar ist.

Überraschenderweise zeigte sich, dass die erfindungsgemäße sehr gut lösliche Calcium-Laktat-Malat Verbindung ausschließlich durch die molare Verhältnisformel $Ca^{2+} (C_3O_3H_5)_x (C_4O_5H_4)_y$ wiedergegeben wird.

Dabei steht in dieser Verbindung $(C_3O_3H_5)^-_x$ für das Laktat mit dem molaren Mengenverhältnis x und $(C_4O_5H_4)^{2-}_y$ für das Malat mit dem molaren Mengenverhältnis y. Die molaren Anteile des Laktats liegen bevorzugt bei x = 1,73 bis x = 1,9 und die entsprechenden molaren Anteile des Malats liegen bevorzugt bei y = 0,135 bis y = 0,05.

Nur bei diesen molaren Verhältnissen ist die Calcium-Laktat-Malat Verbindung sehr gut in wässriger Lösung löslich.

Für die Herstellung der erfindungsgemäßen Calcium-Laktat-Malat Verbindung wird Milchsäure und die Äpfelsäure sowie Calciumcarbonat, Calciumoxid und/oder Calciumhydroxid in vorbestimmten Verhältnissen gelöst. Es werden zunächst die Äpfel- und Milchsäure und erst danach die Calciumverbindung in Lösung gebracht.

Bevorzugt werden äquimolare Verhältnisse von Calcium zu Laktat-Malat-Gemisch durch Überprüfung des pH-Wertes eingestellt.

Alternativ wird Calciumlactat und Calciummalat in einer wässrigen Lösung zu der erfindungsgemäßen Calcium-Lactat-Malat Verbindung umgesetzt.

Nach vollständigem Lösen aller Bestandteile wird der Wasseranteil der Lösung entfernt und es bleibt die reine, schnell lösliche erfindungsgemäße Calcium-Laktat-Malat Verbindung zurück. Übliche Verfahren zum Entfernen des Wassergehaltes sind beispielsweise Sprühtrocknung, Fließbetttrocknung, Walzentrocknung, Wasserentzug in Trockenschränken bzw. Vakuumtrockenschränken, Lyophilisation und andere vergleichbare Verfahren mit oder ohne Anlegen von Unterdruck, sowie Kombinationen hiervon. Die Verfahren werden dabei vorzugsweise so gefahren, dass als Endprodukt ein feines, schnell lösliches Pulver, Granulat oder Lyophilisat vorliegt. Gegebenenfalls werden Zwischenprodukte durch mechanische Verfahren (Walzen, Mühlen, u.ä.) zu einem ausreichend feinen Pulver zerkleinert oder mittels einer Granulations- oder Kompaktierungsanlage zu gröberen Partikeln umgearbeitet.

In einer Ausführungsform wird die Lösung zunächst am Rotationsverdampfer mit Vakuum eingeengt. Dabei werden Temperaturen zwischen ca. 50°C und ca. 100°C, bevorzugt zwischen ca. 70°C und ca. 100°C und besonders bevorzugt zwischen ca. 80°C und ca. 100°C verwendet. Das anliegende Vakuum bewegt sich dabei im Bereich von ca. 1 mbar bis ca. 1000 mbar, bevorzugt zwischen ca. 5 mbar und ca. 100 mbar und besonders bevorzugt zwischen ca. 10 mbar und ca. 50 mbar. Unter diesen Bedingungen wird zunächst soviel Wasser entfernt, dass eine Suspension mit einem Feststoffgehalt von ca. 10 % bis ca. 50 %, bevorzugt von ca. 20 % bis ca. 40 % und besonders bevorzugt von ca. 25 % bis ca. 35 % als Zwischenprodukt vorliegt.

In einer weiter bevorzugten Ausführungsform wird der Wassergehalt und/oder Restwassergehalt in einem Trockenschrank mit Vakuum entfernt. Das anliegende Vakuum bewegt sich dabei im Bereich von ca. 1 mbar bis ca. 1000 mbar, bevorzugt zwischen ca. 5 mbar bis ca. 100 mbar und besonders bevorzugt zwischen ca. 10 mbar bis ca.

50 mbar.

Bei der Trocknung in Trockenschränken mit Unterdruck werden bevorzugt Temperaturen von ca. 20°C bis ca. 95°C, weiter bevorzugt von ca. 40°C bis ca. 90°C und besonders bevorzugt von ca. 50°C bis ca. 80°C verwendet.

Der Vorteil der beschriebenen Herstellungsverfahren liegt darin, dass nach der Trocknung reines Calcium-Laktat-Malat Pulver vorliegt, das unmittelbar mit seinen vorteilhaften Eigenschaften, wie sehr schnelle und gute Löslichkeit, angenehmer Geschmack, Thermostabilität bis 200 °C, hoher Calciumgehalt, zur Supplementierung von Lebensmitteln eingesetzt werden kann.

In einer bevorzugten Ausführungsform des oben beschriebenen Verfahrens wird die wässrige Lösung zum Lösen von Milchsäure und/oder Äpfelsäure erwärmt. Bevorzugt werden dabei Temperaturen von ca. 10°C bis ca. 100°C, besonders bevorzugt von ca. 30°C bis ca. 100°C, weiter bevorzugt von ca. 50°C bis ca. 80°C und besonders bevorzugt ca. 60°C bis ca. 70°C verwendet.

Durch die Erwärmung der Lösung wird insbesondere die Löslichkeit und Lösegeschwindigkeit der im Vergleich zu Milchsäure deutlich schwerer löslichen Äpfelsäure erhöht bzw. des im Vergleich zu Calciumlaktat deutlich schwerer löslichen Calciummalats verbessert.

In einer weiter bevorzugten Ausführungsform wird die Temperatur beim Zugeben der Calciumverbindung, Calciumhydroxide, -oxide und/oder -carbonate, zu der wässrigen Lösung auf ca. 30°C bis ca. 99°C, weiter bevorzugt auf ca. 50°C bis ca. 97°C, besonders bevorzugt auf ca. 85°C bis ca. 95°C, ganz besonders bevorzugt auf ca. 90°C erhöht.

Durch die Erhöhung der Temperatur wird die Lösegeschwindigkeit der Calciumhydroxide, -oxide und/oder -carbonate und gleichzeitig die Gesamtmenge an lösbarem Calcium in einer vorgegebenen Wassermenge deutlich erhöht.

In einer weiteren bevorzugten Ausführungsform wird die Lösung aus Calciummalat und Calciumlaktat erwärmt. Bevorzugt werden dabei Temperaturen von ca. 10°C bis ca. 100°C, besonders bevorzugt von ca. 30°C bis ca. 100°C, weiter bevorzugt von ca. 50°C bis ca. 80°C und besonders bevorzugt ca. 60°C bis ca. 70°C verwendet.

Beide Ausgangsstoffe werden durch die Wärmezufuhr einerseits schneller und andererseits in größerer Menge gelöst.

Die Erhöhung der Menge der gelösten Ausgangsstoffe (Äpfelsäure, Milchsäure, Calciumquelle bzw. Calciumlaktat und Calciummalat) durch die Wärmezufuhr ist ein besonderer Vorteil, da bei dem beschriebenen Herstellungsverfahren das zum Lösen der Ausgangsstoffe notwendige Wasser anschließend vollständig entfernt werden muss. Durch Erhöhung der Temperatur bei den Löseprozessen können die notwendige Wassermenge und damit die entsprechenden Energiekosten, diese später wieder zu entfernen, minimiert werden.

In einer weiteren Ausführungsform wird die Herstellung der Calcium-Laktat-Malat Verbindung unter Ausschluss von Kohlendioxid durchgeführt. Eine Ausführungsform für dieses Herstellungsverfahren ist die Begasung mit Stickstoff.

Der Vorteil des Ausschlusses von Kohlendioxid liegt darin, dass bei Eindringen von Kohlendioxid schlechter lösliches und geschmacklich abweichendes Calciumcarbonat gebildet wird. Dadurch wird die Bildung der gewünschten Calcium-Laktat-Malat Verbindung behindert, es kann zu Ausfällungen kommen, die Gesamtlöslichkeit des Calciums verringert sich und das Herstellungsverfahren ist nicht mehr rückstandsfrei.

Das Ziel eine sehr gut lösliche Calcium-Laktat-Malat Verbindung bereitzustellen, wird stoffseitig durch die Merkmale des Anspruches 8 erfüllt.

Überraschenderweise zeigte sich, dass die Calcium-Laktat-Malat Verbindung ausschließlich durch die folgende molare Verhältnisformel wiedergegeben wird:

$$Ca^{2+} (C_3O_3H_5)_x (C_4O_5H_4)_y.$$

Dabei steht $(C_3O_3H_5)^-_x$ für Laktat mit dem molaren Mengenverhältnis x in dieser Verbindung und $(C_4O_5H_4)^{2-}_y$ für Malat mit dem molaren Mengenverhältnis y. Die molaren Anteile von Laktat liegen bei x = 1,70 bis x = 1,98 und besonders bevorzugt bei x = 1,73 bis x = 1,9 liegen und die entsprechenden molaren Anteile von Malat liegen bei y = 0,15 bis y = 0,01 und besonders bevorzugt bei y = 0,135 bis y = 0,05, wobei jeweils x + 2 y = 2 gilt.

Nur bei diesen molaren Verhältnissen ist die Calcium-Laktat-Malat Verbindung sehr gut in wässriger Lösung löslich und es ergibt sich zudem ein unerwarteter synergistischer Effekt für die Löslichkeit der Verbindung und der sich daraus ergebend Löslichkeit von Calcium. Die Löslichkeit des Calciums aus der Calcium-Laktat-Malat Verbindung, bezogen auf die Einwaage, liegt bei den erfindungsgemäßen molaren Verhältnissen deutlich höher als bei handelsüblichem Calciumlaktat und um ein vielfaches höher als bei Calciummalat.

In einer weiteren Ausführungsform liegt der Calciumgehalt in einem Bereich von 0,8 - 1,1 Mol.

Mit der besonders guten Löslichkeit der Calcium-Laktat-Malat Verbindung mit den erfindungsgemäßen molaren Verhältnissen von Laktat und Malat geht auch eine hohe Lösegeschwindigkeit in wässrigem Medium einher.

Zusätzlich hat die erfindungsgemäße Verbindung eine sehr hohen Calciumgehalt von ca. 18 %, was den Vorteil eines geringen Stoffeinsatzes bei der Anreicherung eines Lebensmittels mit Calcium mit sich bringt.

Die erfindungsgemäße Calcium-Laktat-Malat Verbindung eignet sich besonders gut für die Anreicherung von Lebensmitteln mit Calcium, da sie einen angenehmen Geschmack aufweist.

Die hohe Lösegeschwindigkeit in Kombination mit dem hohen Calciumgehalt der Verbindung führt sowohl zu einer niedrigeren Einsatzmenge der Verbindung als auch zur Zeitersparnis bei der Herstellung von Lebensmitteln.

Die schnelle Löslichkeit ist aber auch ein entscheidender Vorteil im Hinblick auf die Erwartung eines Endverbrauchers, der die erfindungsgemäße Calcium-Laktat-Malat Verbindung zur Supplementierung von Lebensmitteln, insbesondere von flüssigen Lebensmitteln einsetzt.

Die Calcium-Laktat-Malat Verbindung mit den angegebenen Mengenverhältnissen von Laktat und Malat ist ferner unter den für Lebensmittel üblichen thermischen Belastungen sehr stabil. Zudem ist diese Verbindung auch noch bei Temperaturen bis zu 200°C stabil, so dass sie problemlos auch z.B. bei der Pasteurisierung oder Ultrahocherhitzung eingesetzt werden können.

Zudem ist die erfindungsgemäße Verbindung geschmacklich angenehm, was den Einsatz in praktisch allen Lebensmitteln, insbesondere auch in neutralen oder solchen mit geringem Eigengeschmack, ermöglicht.

Auf Grund der hohen Löslichkeit können mit der Calcium-Laktat-Malat Verbindung nicht nur alle flüssigen Lebensmittel, wie Limonaden oder Fruchtsäfte mit einem hohen Calciumgehalt supplementiert werden, sondern auch halbflüssige Lebensmittel wie Joghurt, Quark oder Gelee.

Die ausreichende Löslichkeit des Calciums aus der Calcium-Laktat-Malat Verbindung ist unter anderem Voraussetzung für die Auslobung eines auf diese Weise mit Calcium supplementierten Lebensmittels. Die Verwendung von Calcium darf z. B. in der Europäischen Union derzeit gemäß Richtlinie 90/496/EWG nur ausgelobt werden, wenn die übliche tägliche Verzehrsportion des Lebensmittels mindestens 15 % des RDA enthält (RDA = recommended daily allowance). Beispielsweise können in nur 200 ml Wasser durch eine Calcium-Laktat-Malat Verbindung bis zur 5-fachen Menge des RDA von Calcium ($\cong$ 4 g) gelöst werden, wodurch die Auslobung eines derartigen Lebensmittels möglich wird. Dagegen kann beispielsweise mit handelsüblichem Calcium-Lactat nur die dreifache Menge des RDA in 200 ml Wasser gelöst werden.

Die erfindungsgemäße Calcium-Laktat-Malat Verbindung ist insbesondere für halbfeste und feste Lebensmittel geeignet, da sie auf Grund ihrer guten Lösungseigenschaft nicht nur direkt, sondern auch indirekt in das Lebensmittel eingearbeitet werden kann. Indirekt bedeutet, dass die Verbindung nicht in das fertige Lebensmittel, sondern in ein Zwischenprodukt eingebracht ist. Als Beispiel für ein derartiges Zwischenprodukt kann eine wässrige Lösung genannt werden, wie sie z.B. bei der Herstellung von Pudding oder auch für die Herstellung von Brotteig verwendet werden. Ein weiteres Beispiel für ein Zwischenprodukt ist ein Getränkekonzentrat.

Die Calcium-Laktat-Malat Verbindung ist auf Grund ihrer Geschmacksneutralität und ihres hohen Calciumgehaltes auch gut für die Zubereitung fester Lebensmittel geeignet. In ein festes Lebensmittel kann die Calcium-Laktat-Malat Verbindung direkt, oder, wie bei den halbfesten Lebensmittel beschrieben, indirekt über zum Beispiel eine Flüssigkeit oder auch ein festes Ausgangs- bzw. Zwischenprodukt wie zum Beispiel Mehl eingebracht werden.

In einer weiteren Ausführungsform liegt die Calcium-Laktat-Malat Verbindung in Mischungen mit mindestens einem Hilfsstoff vor. Derartige Hilfsstoffe können beispielsweise Zucker, Zuckeraustauschstoffe, Süßstoffe, Emulagatoren, Farbstoffe, Geschmacksstoffe, Konservierungsmittel, Schäumer, Vitamine, Trennmittel, Pufferungssubstanzen, Säuerungsmittel, Antioxidationsmittel, Stabilisatoren wie zum Beispiel Traganth, Gummi arabicum, Pektin, Gelatine oder Karageen sein.

Die Vorteile einer Mischung mit o. g. Hilfsstoffen sind vielfältig. Sie dienen bekannter Weise der Geschmacksverbesserung, Färbung, Haltbarmachung, Stabilisierung etc.. Insbesondere der Zusatz von Stabilisatoren wird bevorzugt verwendet, um Ausfällungen in zum Beispiel stark gesättigten Lösungen zu verhindern.

Das Ziel ein Lebensmittel mit hohem Calciumgehalt im Hinblick auf seine empfohlene Tagesverzehrsmenge bereitzustellen, dessen Geschmack nicht negativ durch den hohen Mineralstoffgehalt beeinträchtigt wird, wird durch die Merkmale des Anspruches 12 gelöst.

In einer Ausführungsform enthält das Lebensmittel mindestens eine erfindungsgemäße Calcium-Laktat-Malat Verbindung.

In einer weiteren Ausführungsform sind in demselben Lebensmittel neben der Calcium-Laktat-Malat Verbindung andere Calciumderivate und/oder andere Mineralstoffe enthalten.

Gemäß einer bevorzugten Ausführungsform eines Lebensmittels, das mit der erfindungsgemäßen Calcium-Laktat-Malat Verbindung angereichert ist, handelt es sich um ein Getränk. Beispiele für ein derartiges Getränk sind: ein geschmacksneutrales Getränk, ein Getränk auf Fruchtsaftbasis, ein Getränk mit künstlichen und/oder natürlichen bzw. naturidentischen Aromastoffen, ein Getränk auf Milchbasis sowie entsprechende Mischformen. Dieses Lebensmittel kann in einer besonderen Ausführungsform auch verzehrsübliche Mengen an Alkohol enthalten.

In einer weiteren Ausführungsform ist das mit der erfindungsgemäßen Calcium-Laktat-Malat Verbindung angereicherte Lebensmitten ein halbfestes Lebensmittel, d.h. ein Lebensmittel mit einem relativ hohen Wasseranteil, das jedoch nicht mehr flüssig, allenfalls zähflüssig ist. Hierzu zählen auch Lebensmittel mit einem sehr hohen Wasseranteil, die mit Stabilisatoren wie Gelatine, Karageen, Traganth o.ä. verfestigt worden sind.

Als halbflüssige Lebensmittel sind beispielhaft Joghurt, Quarkzubereitungen, Puddings und/oder Gelatinezubereitungen zu nennen.

In flüssigen und halbflüssigen Lebensmitteln kommen alle Vorteile der erfindungsgemäßen Calcium-Laktat-Malat Verbindung zur Geltung. Diese Lebensmittel können mit einem hohen Anteil an Calcium geschmacklich angenehm supplementiert werden. Auf Grund des hohen Mineralstoffanteiles in der Calcium-Laktat-Malat Verbindung werden für die Herstellung nur geringe Mengen dieser Verbindung benötigt. Zudem löst sich die Verbindung wegen ihrer guten Löslichkeit schnell und in ausreichender Menge auf. Dadurch ist es möglich auch in geringen Verzehrsmengen eines Lebensmittels die empfohlene Tagesverzehrsmenge an Calcium zu lösen.

In einer weiteren Ausführungsform handelt es sich bei dem Lebensmittel, das mit der erfindungsgemäßen Calcium-Laktat-Malat Verbindung angereichert ist, um ein Lebensmittel für Personengruppen mit besonderen Anforderungen an die Ernährung. Hierzu zählen beispielsweise Kindernahrung, Sportlernahrung, Nahrung für alte Menschen, Nahrung für kranke Menschen, diätische Lebensmittel bzw. Lebensmittel zur Nahrungsergänzung und/oder Nahrungsergänzungsmittel.

Vorzugsweise ist die erfindungsgemäße Calcium-Laktat-Malat Verbindung Bestandteil eines Nahrungsergänzungsmittels, das als Flüssigkeit, halbfestes Nahrungsergänzungsmittel (z.B. Sirup) oder festes Nahrungsergänzungsmittel (z.B. Tablette, Pulver) vorliegt. Das Nahrungsergänzungsmittel dient seinerseits zum direkten Verzehr und/oder zur Anreicherung von Lebensmitteln mit Calcium.

In einer weiter bevorzugten Ausführungsform orientiert sich der Anteil des Calciums im Lebensmittel, das mit der erfindungsgemäßen Calcium-Laktat-Malat Verbindung angereichert ist und/oder die Gesamtmenge mehrerer Calciumderivate untereinander am Tagesbedarf. In besonderen Ausführungsformen kann sich die Menge des Calciums auch an dem speziellen Bedarf ausgewählter Konsumentengruppen orientieren.

Calcium ist der Mineralstoff, der wegen seiner hohen empfohlenen Tagesverzehrsmengen in großen Mengen in Lebensmittel eingebracht werden soll. Die empfohlene Zufuhr an Calcium liegt bei ca. 800 mg/Tag (RDA) bzw. 1000 mg (DACH-Wert).

In einer bevorzugten Ausführungsform ist eine übliche Tagesverzehrsmenge des mit der erfindungsgemäßen Calcium-Laktat-Malat Verbindung supplementierten Lebensmittels mit ca. 1 % bis ca. 100 %, weiter bevorzugt mit ca. 15 % bis ca. 100 % und besonders bevorzugt mit ca. 50 % bis ca. 100 % des Tagesbedarfes an Calcium angereichert.

In einer weiter bevorzugten Ausführungsform sind die genannten Anteile Calcium am Gesamttagesbedarf in ca. 1 ml bis ca. 1 l, weiter bevorzugt in ca. 50 ml bis ca. 500 ml und besonders bevorzugt in einer Menge von ca. 150 ml bis ca. 250ml, ganz besonders bevorzugt in ca. 200 ml eines flüssigen oder halbflüssigen Lebensmittel gelöst.

Der Vorteil eines Lebensmittels, das mit einem definierten Anteil an Calcium angereichert ist, liegt darin, dass mit diesem Lebensmittel kontrolliert Anteile einer empfohlenen Tagesdosierung dieses Mineralstoffes, bei dem beispielsweise eine Mangelversorgung besteht, aufgenommen werden können.

Gemäß einer Weiterbildung der Erfindung ist dem mit der erfindungsgemäßen Calcium-Laktat-Malat Verbindung angereicherten Lebensmittel mindestens ein weiterer Hilfsstoff mit seinen entsprechenden bekannten vorteilhaften Eigenschaften zugesetzt. Geeignete Hilfsstoffe sind im Zusammenhang mit der Beschreibung der Mineralstoff-Laktat-Malat Verbindung beispielhaft aufgezählt.

In einer bevorzugten Ausführungsform eines flüssigen oder halbflüssigen Lebensmittels mit der erfindungsgemäßen Calcium-Laktat-Malat Verbindung ist das Lebensmittel mit einem Stabilisator versetzt.

Der Vorteil ein mit Calcium supplementiertes Lebensmittel mit einem Stabilisator zu versetzen liegt darin, dass ungewünschte Sedimentierung der Lebensmittelinhaltsstoffe verhindert werden können. Dies ist insbesondere bei z.B. einem Fruchtsaft und/oder Milchmischgetränk vorteilhaft, um auch bei längerem Stehen eine Ausfällung von Proteinen zu verhindern und die Homogenität des Getränkes zu gewährleisten.

Das Ziel, ein Lebensmittel mit einem hohen Mineralstoffanteil und hoher Geschmacksneutralität herzustellen wird durch die Merkmale des Anspruches 15 wiedergegeben.

In einer bevorzugten Ausführungsform wird die erfindungsgemäße Calcium-Laktat-Malat Verbindung vorzugsweise in einem flüssigen Lebensmittel gelöst oder direkt mit einem festen Lebensmittel vermischt.

In einer weiteren bevorzugten Ausführungsform, wie beispielsweise bei einem halbfesten oder festen Lebensmittel, wird mindestens eine Mineralstoff-Laktat-Malat Verbindung bei der Zubereitung in ein flüssiges oder festes Zwischenprodukt gegeben.

Beim Lösen der Calcium-Laktat-Malat Verbindung in einer Flüssigkeit kommen alle seine Vorteile, wie hohe Löslichkeit, schnelle Lösungsgeschwindigkeit, hoher Mineralstoffanteil und Geschmacksneutralität zum tragen.

In einer weiteren Ausführungsform werden die Ausgangsprodukte der Calcium-Laktat-Malat Verbindung, Calciumcarbonat, -oxid und/oder -hydroxid, Milchsäure und Äpfelsäure, alternativ Calciumlaktat und Calciummalat mit ihren vorherbestimmten Anteilen direkt in einem flüssigen Lebensmittel oder Lebensmittelbestandteil, in Anlehnung an das oben für eine wässrige Lösung beschriebene Verfahren, gelöst.

In einer weiter bevorzugten Ausführungsform wird das Lösen durch die Zuführung von Wärme zu dem flüssigen Lebensmittel oder Lebensmittelbestandteil beschleunigt. Unter Berücksichtigung der Beschaffenheit des Lebensmittels werden die Bedingungen analog zu den Bedingungen zum Lösen in wässrigem Medium ausgewählt.

Dieses Verfahren ist für alle Lebensmittel bzw. entsprechende Zwischenprodukte von Vorteil, die beispielsweise unter Wärmezufuhr zu einem Konzentrat eingedickt oder gar getrocknet werden, da sich bei den erhöhten Temperaturen die Ausgangsstoffe Äpfelsäure, Milchsäure und mindestens ein basisches Calciumderivat alternativ das Calciumlaktat und Calciummalat besonders gut lösen. Beim herstellungsgemäßen Verdünnen mit Wasser steht der gewünschte Mineralstoff dann schnell und in hohen Mengen in der Lösung zur Verfügung.

Das Ziel die Calcium-Laktat-Malat Verbindung für die Zubereitung von Lebensmitteln zu verwenden, wird durch die Merkmale des Patentanspruches 19 wiedergegeben.

Die Vorteile die die Calcium-Laktat-Malat Verbindung bei der Verwendung für die Zubereitung von Lebensmitteln hat sind bereits unter den stofflichen Eigenschaften sowie bei der Herstellung von Lebenmitteln mit Calcium-Laktat-Malat Verbindung dargestellt.

Das Ziel die Calcium-Laktat-Malat Verbindung für die Herstellung einer pharmazeutischen Zubereitung zu verwenden wird durch die Merkmale des Patentanspruches 20 wiedergegeben.

Pharmazeutische Zubereitungen umfassen beispielsweise Tabletten, Kapseln, Dragees, Pellets, Pulver Granulate, Suppositorien, Säfte, Sirupe, wässrige und alkoholische Lösungen. Ihnen können übliche Mengen an pharmazeutischen Hilfsstoffen zugesetzt sein.

In einer bevorzugten Ausführungsform wird die erfindungsgemäße Calcium-Laktat-Malat Verbindung für die Herstellung von pharmazeutischen Zubereitungen für die Behandlung der Osteoporose eingesetzt. Hierbei ist es besonders wichtig hohe Mengen an Calcium in der entsprechenden Darreichungsform zu lösen oder zu verarbeiten, was mit der erfindungsgemäßen Calcium-Laktat-Malat Verbindung erreicht wird.

In einigen Ausführungsformen ist die Calcium-Laktat-Malat Verbindung bezogen auf Calcium Mengen in 1 g bis 10 g, 0,5 g bis 1 g, 0,1 g bis 0,5 g oder 0,01 g bis 0,1 g enthalten. Die Angabe bezieht sich die Gesamtmenge einer Darreichungsform, die üblicherweise an einem Tag appliziert wird.

In einigen Ausführungsformen ist die erfindungsgemäße Calcium-Laktat-Malat Verbindung bezogen auf die täglich zugeführte Calcium-Menge in geringeren Mengen als 0,01 g enthalten, vereinzelt auch in höheren Mengen als 10g.

Die Vorteile die erfindungsgemäße Calcium-Laktat-Malat Verbindung für die Herstellung einer pharmazeutischen Zubereitung zu verwenden liegt in ihren vorteilhaften Eigenschaften. Analog der Herstellung von Lebensmitteln kann auf Grund des hohen Calciumgehaltes in einer geringen Menge sehr viel Calcium verabreicht werden. Der angenehme Geschmack der erfindungsgemäßen Calcium-Laktat-Malat Verbindung ermöglicht den Einsatz der Calcium-Laktat-Malat Verbindung nicht nur in Tabletten, Kapseln, Pellets, Dragees, die geschluckt werden, sondern auch in Darreichungsformen wie Säften, Sirups, Lutschtabletten, Trinkkonzentraten, Pulvern etc. bei deren Aufnahme der Geschmack eine entscheidende Rolle spielt.

Durch die hohe Lösungsgeschwindigkeit der Calcium-Laktat-Malat Verbindung eignet sie sich insbesondere auch für die Herstellung von schnelllöslichen Trockensubstanzen wie Brausetabletten, Pulvern oder Granulaten für die Zubereitung von trinkfertigen Darreichungsformen.

Ein weiterer Vorteil für die Herstellung von pharmazeutischen Zubereitungen ist die Hitzebeständigkeit der Calcium-Laktat-Malat Verbindung bis 200 °C, die es ermöglicht die erfindungsgemäße Calcium-Laktat-Malat Verbindung auch für Produkte, die einer Hitzesterilisation ausgesetzt werden müssen, wie beispielsweise Infusionslösungen, zu verwenden.

**Beispiele**

1. Beispiel

Herstellung der Calcium-Laktat-Malat Verbindung mit der Formel:

$$Ca(C_3O_3H_5)_{1,73}(C_4O_5H_4)_{0,135}$$

1,73 Mol Milchsäure werden unter Erwärmen und Rühren in 500 ml Wasser gegeben und danach mit 0,135 Mol Äpfelsäure ebenfalls unter Erwärmen und Rühren innerhalb von 20 min aufgelöst. Anschließend wird die Lösung in 10 min auf 90°C erwärmt. Bei dieser Temperatur wird 1 Mol Calciumhydroxid innerhalb von 30 min langsam zur Lösung unter intensivem Rühren hinzugegeben, bis ein pH-Wert von 5,5 erreicht ist.

Die Lösung wird am Rotationsverdampfer im Vakuum von 20 mbar bis zu einem Feststoffgehalt von max. 30% oberhalb von 80°C eingeengt. Anschließend wird die Lösung im Vakuumtrockenschrank (z.B. Wasserstrahlpumpenvakuum) bei 70°C eingedampft und getrocknet. Man erhält in 100%iger Ausbeute eine sehr gut lösliche Calcium-Laktat-Malat Verbindung.

2. Beispiel

Herstellung der Calcium-Laktat-Malat Verbindung mit der Formel:

$$Ca(C_3O_3H_5)_{1,86}(C_4O_5H_4)_{0,07}$$

0,07 Mol Calciummalat werden in 2000 ml Wasser gegeben. Die Suspension wird auf 90°C erhitzt. Anschließend werden 1,86 Mol Calciumlaktat hinzugefügt. Der Ansatz wird 30 Minuten bei einer Temperatur von 90°C gerührt. Der Ansatz wird klar.

Die Lösung wird am Rotationsverdampfer im Vakuum von 20 mbar bis zu einem Feststoffgehalt von max. 30% oberhalb von 80°C eingeengt. Anschließend wird die Lösung im Vakuumtrockenschrank (z.B. Wasserstrahlpumpenvakuum) bei 70°C eingedampft und getrocknet. Man erhält in 100%iger Ausbeute eine sehr gut lösliche Calcium-Laktat-Malat Verbindung.

3. Beispiel

Vergleichsverbindungen 1 bis 4

Eine Menge von x Mol Milchsäure werden unter Erwärmen und Rühren in 500 ml Wasser gegeben und danach mit y Mol Apfelsäure ebenfalls unter Erwärmen und Rühren innerhalb von 20 min aufgelöst. Anschließend wird die Lösung in 10 min auf 90°C erwärmt. Bei dieser Temperatur wird 1 Mol Calciumhydroxid innerhalb von 30 min langsam zur Lösung unter intensivem Rühren hinzu gegeben, bis ein pH-Wert von 5,5 erreicht ist.

Die Lösung wird am Rotationsverdampfer im Vakuum von 20 mbar bis zu einem Feststoffgehalt von max. 30% oberhalb von 80°C eingeengt. Anschließend wird die Lösung im Vakuumtrockenschrank (z.B. Wasserstrahlpumpenvakuum) bei 70°C eingedampft und getrocknet. Man erhält in 100%iger Ausbeute die entsprechende Calcium-Laktat-Malat Verbindung.

| | | |
|---|---|---|
| Vergleichsverbindung 1 | x = 1,48 | y = 0,26 |
| Vergleichsverbindung 2 | x = 1,30 | y = 0,35 |
| Vergleichsverbindung 3 | x = 1,03 | y = 0,48 |
| Vergleichsverbindung 4 | x = 0,92 | y = 0,54 |

4. Beispiel

Vergleichsverbindung 5 bis 7

Herstellung von Calcium-Laktat-Malat $[Ca(C_3O_3H_5)_x(C_4O_5H_4)_y]$

Eine Menge von y Mol Calciummalat wird in 2000 ml Wasser gegeben. Die Suspension wird auf 90°C erhitzt. Anschließend werden x Mol Calciumlaktat hinzugefügt. Der Ansatz wird 30 Minuten bei einer Temperatur von 90°C gerührt. Der Ansatz wird nicht klar.

Die Suspension wird am Rotationsverdampfer im Vakuum von 20 mbar bis zu einem Feststoffgehalt von max. 30% oberhalb von 80°C eingeengt. Anschließend wird die Lösung im Vakuumtrockenschrank (z.B. Wasserstrahlpumpenvakuum) bei 70°C eingedampft und getrocknet.

| | | |
|---|---|---|
| Vergleichsverbindung 5 | x = 1,40 | y = 0,26 |
| Vergleichsverbindung 6 | x = 1,30 | y = 0,35 |
| Vergleichsverbindung 7 | x = 1,03 | y = 0,48 |
| Vergleichsverbindung 8 | x = 0,92 | y = 0,54 |

5. Beispiel

Bestimmung des löslichen Calcium Anteils in Calcium-Verbindungen

Jeweils 10 g (genau gewogen) des Calcium-Laktat-Malats, Calciumlaktats bzw. des Calciummalats werden in 100 g Wasser suspendiert und 30 min bei Raumtemperatur auf einem Magnetrührer gerührt. Anschließend wird die Suspension über einen Faltenfilter filtriert. 2 g des Klarfiltrats, genau gewogen, werden in einem 200 ml Erlenmeierkolben eingewogen. 2 ml HCl 16%, 10 ml Puffer Lösung pH 10 und eine Spatelspitze Magnesium-Komplexoat werden hinzugegeben. Es wird mit 0,1 N Titriplex III Lösung gegen Eriochromschwarz von rot nach blau filtriert.

Berechnung:

$$\frac{4{,}008 \times \text{Verbrauch} \times \text{Titer Lösung}}{\text{Einwaage} \times 100} = \text{Ca / g Einwaage}$$

Gemäß Beispiel 2 wurde der Anteil des löslichen Calciums der Calcium-Laktat-Malat Verbindung in Abhängigkeit des Verhältnisses von Laktat zu Malat bestimmt. Als Referenz wurde die Löslichkeit von Calcium aus reinem Calcium-Laktat bzw. reinem Calciummalat gemäß nachfolgender Tabelle 1 bestimmt.

Tabelle 1 Die Tabelle 1 gibt den Anteil des gelösten Calciums in Prozent bezogen auf die Einwaage der verschiedenen Calciumverbindungen an.

| Beispiel | Calciumsalz | Molare Zusammensetzung | Anteil lösliches Calcium am Gesamtcalcium [%] |
|---|---|---|---|
| | Calcium-Laktat-Malat | $Ca (C_3O_3H_5)_{1,90} (C_4O_5H_4)_{0,05}$ | 99,8 |
| Beispiel 2 | Calcium-Laktat-Malat | $Ca (C_3O_3H_5)_{1,86} (C_4O_5H_4)_{0,07}$ | 100,0 |
| Beispiel 1 | Calcium-Laktat-Malat | $Ca (C_3O_3H_5)_{1,73} (C_4O_5H_4)_{0,135}$ | 100,0 |
| Vergleichsverbindung 1 | Calcium-Laktat-Malat | $Ca (C_3O_3H_5)_{1,48} (C_4O_5H_4)_{0,26}$ | 82,7 |
| Vergleichsverbindung 2 | Calcium-Laktat-Malat | $Ca (C_3O_3H_5)_{1,30} (C_4O_5H_4)_{0,35}$ | 71,4 |
| Vergleichsverbindung 3 | Calcium-Laktat-Malat | $Ca (C_3O_3H_5)_{1,03} (C_4O_5H_4)_{0,48}$ | 66,0 |
| Vergleichsverbindung 4 | Calcium-Laktat-Malat | $Ca (C_3O_3H_5)_{0,92} (C_4O_5H_4)_{0,54}$ | 47,8 |
| Vergleichsverbindung 5 | Calcium-Laktat-Malat | $Ca (C_3O_3H_5)_{1,48} (C_4O_5H_4)_{0,26}$ | 80,4 |
| Vergleichsverbindung 6 | Calcium-Laktat-Malat | $Ca (C_3O_3H_5)_{1,30} (C_4O_5H_4)_{0,35}$ | 69,3 |
| Vergleichsverbindung 7 | Calcium-Laktat-Malat | $Ca (C_3O_3H_5)_{1,03} (C_4O_5H_4)_{0,48}$ | 57,8 |
| Vergleichsverbindung 8 | Calcium-Laktat-Malat | $Ca (C_3O_3H_5)_{0,92} (C_4O_5H_4)_{0,54}$ | 43,1 |
| Kommerziell erhältlich | Calciumlaktat E 327 | $Ca (C_3O_3H_5)_2$ | 83,0 |
| Kommerziell erhältlich | Calciummalat E 352 | $Ca (C_4O_5H_4)$ | 3,6 |

Vorteile und Zweckmäßigkeit des Calcium-Laktat-Malat Verbindung sind der nachfolgenden Beschreibung in Verbindung mit der einzigen Figur zu entnehmen. Diese zeigt eine Darstellung des Anteils an löslichem Calcium in einer Calcium-Laktat-Malat Verbindung in Abhängigkeit der verschiedenen molaren Anteile an Laktat und Malat sowie von reinem Calcium-lactat bzw. reinem Calciummalat.

Die Bestimmung des freien Calciums erfolgte gemäß dem im Beispiel 5 geschilderten Verfahren.

In der Figur sind auf der y-Achse prozentual die Anteile an Calcium aufgetragen, die sich aus einer Calciumverbindung lösen. Die Balken stellen von links nach rechts die molaren Verhältnisse von Laktat und Malat dar, wie sie in folgender Tabelle wiedergegeben sind (Balken 1 - 7). Als Referenzen sind die Anteile an löslichem Calcium bei reinem Calcium-Laktat (Balken 8) bzw. reinem Calciummalat (Balken 9) dargestellt.

Die Abbildung zeigt, dass bei den molaren Anteilen von Laktat zwischen 1,73 und 1,90 und den korrespondierenden molaren Anteilen von Malat zwischen 0,135 und 0,05 die Löslichkeit des Mineralstoffes Calcium praktischen 100% beträgt.

6. Beispiel

In Tabelle 2 sind die Eigenschaften der Calcium-Laktat-Malat Verbindung im Vergleich zu Calcium Verbindungen aus dem Stand der Technik dargestellt:

Tabelle 2

| Calciumsalz | Löslichkeit 10 g In 100 ml $H_2O$ | Calcium Gehalt | In 10 g Verbindung enthaltenes Calcium [mg] | Einsatzmenge Calciumsalz für ½ RDA* (400 mg Ca) | Schnelle Löslichkeit in Wasser 10g in 100 ml | Thermostabilität >140°C | Guter Geschmack |
|---|---|---|---|---|---|---|---|
| Calcium-Laktat-Malat $Ca(C_3O_3H_5)_x$ $(C_4O_5H_4)_y$ X = 1,73-1,90 Y = 0,135-0,05 | **X** | **X (18,1%)** | **1810** | **2,2 g** | **X** | **X** | **X** |
| Calciumacetat | X | X (24,0%) | 2400 | 1,7 g | X | X | - |
| Calciumcarbonat | - | X (40,0%) | 4000 | 1,0 g | - | X | - |
| Calciumcitrat | - | X (21,0%) | 2100 | 1,9 g | - | - | X |
| Calciumglugonat | - | - (9,00%) | 900 | 4,4g | - | - | X |
| Calciumlactat | - | - (13,5%) | 1370 | 3,7 g | - | X | X |
| Calciulactogluconat | X | - (10,2%) | 1020 | 4,9 g | X | - | X |
| Calciummalat | - | X (23,00%) | 2300 | 1,74 g | - | x | X |

* RDA EU 800 mg Calcium gemäß Richtlinie 90/496/EWG
X trifft zu
- trifft nicht zu

Überraschenderweise ergibt sich nur bei den molaren Verhältnissen von x = 1,73 - 1,9 sowie y = 0,05 - 0,135 ein synergistischer Effekt in der Löslichkeit im Vergleich zum handelsüblichen Calciumlaktat bzw. Calciummalat.

Diese Beobachtung wird durch folgende Untersuchungen gestützt:

a) Die Löslichkeit von Calcium ist bei der Calcium-Laktat-Malat Verbindung $[Ca(C_3O_3H_5)_x (C_4O_5H_4)_y]$ im Bereich von x = 1,73 - 1,9 und y = 0,135 - 0,05 um ca. 55 % besser als beim handelsüblichen Calciumlaktat (= E327, siehe Tabelle 2). In Tabelle 3 ist die Löslichkeit von Calcium-Laktat-Malat im Vergleich mit der Löslichkeit von Calciumlaktat bei 25°C dargestellt.

Tabelle 3:

| Calciumsalz | Calciumgehalt [%] | Löslichkeit in 100 ml [g] | Gelöstes Ca [mg] | Faktor |
|---|---|---|---|---|
| Calciumlaktat 4,5 Hydrat E 327 Purac CH622582 | 13,61 | 9,75 | 1327 | 1,549 |
| Calcium-Laktat-Malat DPL CH 122861 | 17,87 | 11,5 | 2055 | 1 |

b) Die Überprüfung des Filtrates nach Beispiel 3 durch HPLC-Untersuchung ergibt unabhängig von der eingesetzten Menge an Calcium, Laktat und Malat eine Zusammensetzung der sich im Filtrat gelösten Calcium-Laktat-Malat Verbindung $[Ca(C_3O_3H_5)_x(C_4O_5H_4)_y]$ nur im Bereich von x = 1,73 - 1,90 und y = 0,135 - 0,05 mit der Bedingung x+ 2 y = 2. Demzufolge bildet nur eine Calcium-Laktat-Malat mit dieser Zusammensetzung die erfindungsgemäße sehr gut lösliche Calciumverbindung.

c) Ebenso führt die Reaktion von Calciumlaktat mit Calciummalat dann zu einer gut löslichen Verbindung, wenn man Calciumlaktat und Calciummalat wie in Beispiel 2 in einem solchen Verhältnis mischt und in $H_2O$ reagieren lässt, dass die molaren Anteile von Laktat in der Calcium-Laktat-Malat Verbindung im Bereich von x = 1,73 - 1,90 liegen, die molaren Anteile von Malat in einem Bereich von y = 0,135-0,05 liegen und x + 2y = 2 gilt.

Das ansonsten kaum lösliche Calciummalat löst sich bei dieser Reaktionsführung komplett zusammen mit dem Calciumlaktat auf. Die resultierende Calciumlaktat-Malat Verbindung zeigt eine sehr gute Löslichkeit.

Sämtliche in den Anmeldungsunterlagen offenbarte Merkmale sind als erfindungswesentliche Merkmal anzusehen. Abwandlungen hiervon sind dem Fachmann geläufig.

**Bezugszeichenliste**

1     Calcium-Laktat-Malat Verbindung mit 0,92 Mol Anteilen Laktat und 0,54 Mol-Anteilen Malat
2     Calcium-Laktat-Malat Verbindung mit 1,03 Mol Anteilen Laktat und 0,48 Mol-Anteilen Malat
3     Calcium-Laktat-Malat Verbindung mit 1,30 Mol Anteilen Laktat und 0,35 Mol-Anteilen Malat
4     Calcium-Laktat-Malat Verbindung mit 1,48 Mol Anteilen Laktat und 0,26 Mol-Anteilen Malat
5     Calcium-Laktat-Malat Verbindung mit 1,73 Mol Anteilen Laktat und 0,135 Mol-Anteilen Malat
6     Calcium-Laktat-Malat Verbindung mit 1,86 Mol Anteilen Laktat und 0,07 Mol-Anteilen Malat
7     Calcium-Laktat-Malat Verbindung mit 1,90 Mol Anteilen Laktat und 0,05 Mol-Anteilen Malat
8     Calcium-Laktat
9     Calcium-Malat

**Patentansprüche**

1.  Verfahren zur Herstellung eines Calcium-Derivates,
    **gekennzeichnet durch** folgende Schritte:

    - Lösen vorbestimmter Anteile an Milchsäure und Äpfelsäure in Wasser zur Bildung einer Lösung;
    - Zugeben vorbestimmter Anteile an Calciumhydroxid, Calciumcarbonat und/oder Calciumoxid zu der Lösung und
    - Entfernen des Wasseranteils der Lösung zur vollständigen Umsetzung der vorbestimmten Anteile zu einer Calcium- Laktat- Malat Verbindung mit der folgenden Verhältnisformel: $Ca^{2+} (C_3O_3H_5)^{-}_x (C_4O_5H_4)^{2-}_y$,

wobei x und y die molaren Verhältnisse von Laktat bzw. Malat bezogen auf 1 Mol Calcium in dieser Calcium-Laktat-Malat Verbindung angeben,

$$x + 2\,y = 2\ \text{gilt,}$$

der molare Anteil des Laktats in einem Bereich von x = 1,7 - 1,98 liegt und der molare Anteil des Malats in einem Bereich von y = 0,15 - 0,01 liegt.

2. Verfahren nach Anspruch 1,
**dadurch gekennzeichnet, dass**
der molare Anteil des Laktats in einem Bereich von x = 1,73 - 1,9 liegt und der molare Anteil des Malats in einem Bereich von y = 0,135 - 0,05 liegt.

3. Das Verfahren nach Anspruch 1 oder 2,
**dadurch gekennzeichnet, dass**
die Lösung beim Lösen von Milchsäure und/oder Äpfelsäure und/oder beim Lösen von Calciumhydroxid, Calciumcarbonat und/oder Calciumoxid
zusätzlich auf eine vorherbestimmbare Temperatur erwärmt wird.

4. Verfahren zur Herstellung eines Calcium-Derivates,
**gekennzeichnet durch folgende Schritte:**

- Lösen vorbestimmter Anteile von Calicumlaktat und Calciummalat in Wasser zu einer Lösung und
- Entfernen des Wasseranteils der Lösung zur vollständigen Umsetzung der vorbestimmten Anteile zu einer Calcium- Laktat- Malat Verbindung mit der folgenden Verhältnisformel: $Ca^{2+}\,(C_3O_3H_5)^-_x\,(C_4O_5H_4)^{2-}_y$,

wobei x und y die molaren Verhältnisse von Laktat bzw. Malat bezogen auf 1 Mol Calcium in dieser Calcium-Laktat-Malat Verbindung angeben,

$$x + 2\,y = 2\ \text{gilt,}$$

der molare Anteil des Laktats in einem Bereich von x = 1,7 - 1,98 liegt und der molare Anteil des Malats in einem Bereich von y = 0,15 - 0,01 liegt.

5. Verfahren nach Anspruch 4,
**dadurch gekennzeichnet, dass**
der molare Anteil von Laktat in einem Bereich von x = 1,73 - 1,9 liegt und der molare Anteil des Malats in einem Bereich von y = 0,135 - 0,05 liegt.

6. Verfahren nach Anspruch 4 oder 5,
**dadurch gekennzeichnet, dass**
die Lösung beim Lösen von Calciumlaktat und/oder Calciummalat zusätzlich auf eine vorherbestimmbare Temperatur erwärmt wird.

7. Verfahren nach einem der vorangegangenen Ansprüche,
**dadurch gekennzeichnet, dass**
der Calciumanteil in der Calcium-Laktat-Malat Verbindung in einem Bereich von 0,8 - 1,1 Mol liegt.

8. Calcium-Laktat-Malat Verbindung,
**dadurch gekennzeichnet, dass**

- die Calcium-Laktat-Malat-Verbindung durch folgende molare Verhältnisformel wiedergegeben wird: $Ca^{2+}\,(C_3O_3H_5)^-_x\,(C_4O_5H_4)^{2-}_y$,
- wobei x und y die molaren Verhältnisse von Laktat $(C_3O_3H_5)^-$ bzw. Malat $(C_4O_5H_4)^{2-}$ bezogen auf 1 Mol Calcium in dieser Calcium-Laktat-Malat Verbindung angeben,

-

$$x + 2y = 2 \text{ gilt,}$$

- der molare Anteil des Laktats in einem Bereich von x = 1,7 - 1,98 liegt und
- der molare Anteil des Malats in einem Bereich von y = 0,15 - 0,01 liegt.

9. Calcium-Laktat-Malat Verbindung nach Anspruch 8,
**dadurch gekennzeichnet, dass**
der molare Anteil des Laktats in einem Bereich von x = 1,73 - 1,9 liegt und der molare Anteil des Malats in einem Bereich von y = 0,135 - 0,05 liegt.

10. Calcium-Laktat-Malat Verbindung nach Anspruch 8 oder 9,
**dadurch gekennzeichnet, dass**
zusätzlich mindestens einen Hilfsstoff enthalten ist.

11. Calcium-Laktat-Malat Verbindung nach einem der Ansprüche 8 - 10,
**dadurch gekennzeichnet, dass**
ein Calciumanteil in der Calcium-Laktat-Malat Verbindung in einem Bereich von 0,8 - 1,1 Mol liegt.

12. Lebensmittel, angereichert mit Calcium, das mindestens eine Calcium-Laktat-Malat Verbindung nach einem der Ansprüche 8 bis 11 enthält.

13. Lebensmittel nach Anspruch 12,
**dadurch gekennzeichnet, dass**
es Calcium in einer Tagesverzehrsmenge von 15 % bis 100 % des empfohlenen Tagesbedarfes enthält.

14. Lebensmittel nach Anspruch 12 oder 13,
**dadurch gekennzeichnet, dass**
es zusätzlich mindestens einen Hilfsstoff enthält.

15. Verfahren zur Herstellung eines mit mindestens einer Calcium-Laktat-Malat Verbindung angereicherten Lebensmittel nach einem der Ansprüche 12 - 14,
**dadurch gekennzeichnet, dass**
die Calcium-Laktat-Malat Verbindung dem Lebensmittel und/oder mindestens einem Zwischenprodukt zugegeben wird.

16. Verfahren nach Anspruch 15,
**dadurch gekennzeichnet, dass**
vorbestimmte Anteile an Milchsäure und Äpfelsäure sowie Anteile an Calciumhydroxid, Calciumoxid und/oder Calciumcarbonat in das flüssig vorliegende Lebensmittel und/oder das flüssig vorliegende Zwischenprodukt des Lebensmittels gegeben werden.

17. Verfahren nach Anspruch 15 oder 16,
**dadurch gekennzeichnet, dass**
vorbestimmte Anteile an Calciumlaktat und Calciummalat in das flüssig vorliegende Lebensmittel und/oder das flüssig vorliegende Zwischenprodukt des Lebensmittels gegeben werden.

18. Verfahren nach Anspruch 16 oder 17
**dadurch gekennzeichnet, dass**
das flüssig vorliegende Lebensmittel und/oder das flüssig vorliegende Zwischenprodukt bei der Zugabe der Anteile von Milchsäure und/oder Äpfelsäure und/oder bei der Zugabe der Anteile an Calciumhydroxid, Calciumoxid und/oder Calciumcarbonat auf eine vorbestimmbare Temperatur erwärmt wird.

19. Verwendung einer Calcium-Laktat-Malat Verbindung gemäß einem der Ansprüche 8 - 11,
**dadurch gekennzeichnet, dass**

die Calcium-Laktat-Malat-Verbindung einem Lebensmittel zugegeben wird.

20. Verwendung einer Calcium-Laktat-Malat Verbindung gemäß einem der Ansprüche 8 - 11,
**dadurch gekennzeichnet, dass**
die Calcium-Laktat-Malat Verbindung für die Herstellung einer pharmazeutischen Zubereitung verwendet wird.

21. Verwendung der Calcium-Laktat-Malat Verbindung gemäß Anspruch 20,
**dadurch gekennzeichnet, dass**
die pharmazeutische Zubereitung für die Behandlung von Osteoporose verwendet wird.

**Claims**

1. A method of producing a calcium derivative, **characterized by** the following steps:

   - dissolving pre-determined portions of lactic acid and malic acid in water in order to form a solution;
   - adding pre-determined portions of calcium hydroxide, calcium carbonate and/or calcium oxide to the solution, and
   - removing the water portion of the solution for the complete conversion of the pre-determined portions to form a calcium / lactate / malate compound with the following ratio formula: $Ca^{2+} (C_3O_3H_5)^-_x (C_4O_5H_4)^{2-}_y$,
   in which x and y indicate the molar ratios of lactate and malate respectively with respect to 1 mol of calcium in this calcium / lactate / malate compound,

$$x + 2y = 2,$$

   the molar portion of the lactate is in a range of

$$x = 1.7 - 1.98,$$

   ,
   and the molar portion of the malate is in a range of

$$y = 0.15 - 0.01.$$

2. A method according to claim 1, **characterized in that** the molar portion of the lactate is in a range of x = 1.73 - 1.9, and the molar portion of the malate is in a range of y = 0.135 - 0.05.

3. A method according to claim 1 or 2, **characterized in that** during the dissolving of lactic acid and/or malic acid and/or during the dissolving of calcium hydroxide, calcium carbonate and/or calcium oxide the solution is additionally heated to a temperature capable of being pre-determined.

4. A method of producing a calcium derivative, **characterized by** the following steps:

   - dissolving pre-determined portions of calcium lactate and calcium malate in water in order to form a solution, and
   - removing the water portion of the solution for the complete conversion of the pre- determined portions to form a calcium / lactate / malate compound with the following ratio formula: $Ca^{2+} (C_3O_3H_5)^-_x (C_4O_5H_4)^{2-}_y$,

   in which x and y indicate the molar ratios of lactate and malate respectively with respect to 1 mol of calcium in this calcium / lactate / malate compound,

$$x + 2y = 2,$$

the molar portion of the lactate is in a range of

$$x = 1.7 - 1.98,$$

and the molar portion of the malate is in a range of

$$y = 0.15 - 0.01.$$

5. A method according to claim 4, **characterized in that** the molar portion of lactate is in a range of x = 1.73 - 1.9, and the molar portion of the malate is in a range of y = 0.135 - 0.05.

6. A method according to claim 4 or 5, **characterized in that** during the dissolving of calcium lactate and/or calcium malate the solution is additionally heated to a temperature capable of being pre-determined.

7. A method according to any one of the preceding claims, **characterized in that** the calcium portion in the calcium / lactate / malate compound is in a range of 0.8 - 1.1 mol.

8. A calcium / lactate / malate compound, **characterized in that**

   - the calcium / lactate / malate compound is indicated by the following ratio formula:

     $$Ca^{2+} (C_3O_3H_5)^-_x (C_4O_5H_4)^{2-}_y,$$

   - in which x and y indicate the molar ratios of lactate $(C_3O_3H_5)^-$ and malate $(C_4O_5H_4)^{2-}$ respectively with respect to 1 mol of calcium in this calcium / lactate / malate compound,
   -

     $$x + 2y = 2,$$

   - the molar portion of the lactate is in a range of x = 1.7 - 1.98, and
   - the molar portion of the malate is in a range of y = 0.15 - 0.01.

9. A calcium / lactate / malate compound according to claim 8, **characterized in that** the molar portion of the lactate is in a range of x = 1.73 - 1.9, and the molar portion of the malate is in a range of y = 0.135 - 0.05.

10. A calcium / lactate / malate compound according to claim 8 or 9, **characterized in that** at least one auxiliary material is additionally contained.

11. A calcium / lactate / malate compound according to any one of claims 8 to 10, **characterized in that** the calcium portion in the calcium / lactate / malate compound is in a range of 0.8 - 1.1 mol.

12. A food enriched with calcium, which contains at least one calcium / lactate / malate compound according to any one of claims 8 to 11.

13. A food according to claim 12, **characterized in that** it contains calcium in a daily consumption quantity of from 15 % to 100 % of the recommended daily requirement.

14. A food according to claim 12 or 13, **characterized in that** it additionally contains at least one auxiliary material.

15. A method of producing a food enriched with at least one calcium / lactate / malate compound according to any one of claims 12 to 14, **characterized in that** the calcium / lactate / malate compound is added to the food and/or at least one intermediate product.

16. A method according to claim 15, **characterized in that** pre-determined portions of lactic acid and malic acid and portions of calcium hydroxide, calcium oxide and/ or calcium carbonate are added to the food present in the form of a liquid and/or to the intermediate product of the food present in the form of a liquid.

17. A method according to claim 15 or 16, **characterized in that** pre-determined portions of calcium lactate and calcium malate are added to the food present in the form of a liquid and/or to the intermediate product of the food present in the form of a liquid.

18. A method according to claim 16 or 17, **characterized in that** during the addition of the portions of lactic acid and/or malic acid and/or during the addition of the portions of calcium hydroxide, calcium oxide and/ or calcium carbonate the food present in the form of a liquid and/or the intermediate product present in the form of a liquid is or are heated to a temperature capable of being pre-determined.

19. Use of a calcium / lactate / malate compound according to any one of claims 8 to 11, **characterized in that** the calcium / lactate / malate compound is added to a food.

20. Use of a calcium / lactate / malate compound according to any one of claims 8 to 11, **characterized in that** the calcium / lactate / malate compound is used for producing a pharmaceutical preparation.

21. Use of a calcium / lactate / malate compound according to claim 20, **characterized in that** the pharmaceutical preparation is used for the treatment of osteoporosis.

**Revendications**

1. Procédé pour la fabrication d'un dérivé de calcium,
   **caractérisé par** les étapes suivantes consistant à :

   - dissoudre des parties prédéterminées d'acide lactique et d'acide malique dans de l'eau pour former une solution ;
   - additionner des parties prédéterminées d'hydroxyde de calcium, de carbonate de calcium et/ou d'oxyde de calcium à la solution, et
   - éliminer la partie d'eau de la solution pour la transformation complète des parties prédéterminées en un composé de lactate-malate de calcium répondant à la formule brute suivante : $Ca^{2+}(C_3O_3H_5)^-_x(C_4O_5H_4)^{2-}_y$,

   dans laquelle x et y indiquent les rapports molaires du lactate ou du malate pour 1 mole de calcium dans ce composé de lactate-malate de calcium,
   s'applique x + 2 y = 2,
   la fraction molaire du lactate se situe dans une plage de x = 1,7 à 1,98 et la fraction molaire du malate se situe dans une plage de y = 0,15 à 0,01.

2. Procédé selon la revendication 1,
   **caractérisé en ce que**
   la fraction molaire du lactate se situe dans une plage de x = 1,73 à 1,9 et **en ce que** la fraction molaire du malate se situe dans une plage de y = 0,135 à 0,05.

3. Procédé selon la revendication 1 ou 2,
   **caractérisé en ce que**
   la solution est en outre chauffée, lors de la dissolution d'acide lactique et/ou d'acide malique et/ou lors de la dissolution d'hydroxyde de calcium, de carbonate de calcium et/ou d'oxyde de calcium, à une température à prédéterminer préalablement.

4. Procédé pour la fabrication d'un dérivé de calcium,
   **caractérisé par** les étapes suivantes consistant à :

   - dissoudre des parties prédéterminées de lactate de calcium et de malate de calcium dans de l'eau pour obtenir une solution, et
   - éliminer la partie d'eau de la solution pour la transformation complète des parties prédéterminées en un composé de lactate-malate de calcium répondant à la formule brute suivante : $Ca^{2+}(C_3O_3H_5)^-_x(C_4O_5H_4)^{2-}_y$,

dans laquelle x et y indique les rapports molaires du lactate ou du malate pour 1 mole de calcium dans ce composé de lactate-malate de calcium,
s'applique x + 2 y = 2,
la fraction molaire du lactate se situe dans une plage de x = 1,7 à 1,98 et la fraction molaire du malate se situe dans une plage de y = 0,15 à 0,01.

**5.** Procédé selon la revendication 4,
**caractérisé en ce que**
la fraction molaire du lactate se situe dans une plage de x = 1,73 à 1,9 et **en ce que** la fraction molaire du malate se situe dans une plage de y = 0,135 à 0,05.

**6.** Procédé selon la revendication 4 ou 5,
**caractérisé en ce que**
la solution est en outre chauffée, lors de la dissolution de lactate de calcium et/ou de malade de calcium, à une température à prédéterminer préalablement.

**7.** Procédé selon l'une quelconque des revendications précédentes, **caractérisé en ce que**
la fraction de calcium dans le composé de lactate-malate de calcium se situe dans une plage de 0,8 à 1,1 mole.

**8.** Composé de lactate-malate de calcium,
**caractérisé en ce que**
le composé de lactate-malate de calcium est représenté par la formule brute molaire suivante : $Ca^{2+}(C_3O_3H_5)^-_x$ $(C_4O_5H_4)^{2-}_y$,
dans laquelle x et y indiquent les rapports molaires du lactate $(C_3O_3H_5)^-$ ou du malate $(C_4O_5H_4)^{2-}$ pour 1 mole de calcium dans ce composé de lactate-malate de calcium,
s'applique x + 2 y = 2,
la fraction molaire du lactate se situe dans une plage de x = 1,7 à 1,98 et la fraction molaire du malate se situe dans une plage de y = 0,15 à 0,01.

**9.** Composé de lactate-malate de calcium selon la revendication 8,
**caractérisé en ce que**
la fraction molaire du lactate se situe dans une plage de x = 1,73 à 1,9 et **en ce que** la fraction molaire du malate se situe dans une plage de y = 0,135 à 0,05.

**10.** Composé de lactate-malate de calcium selon la revendication 8 ou 9,
**caractérisé en ce que**
ce dernier contient en outre au moins un adjuvant.

**11.** Composé de lactate-malate de calcium selon l'une quelconque des revendications 8 à 10,
**caractérisé en ce que**
une fraction de calcium dans le composé de lactate-malate de calcium se situe dans une plage de 0,8 à 1,1 mole.

**12.** Produit alimentaire, enrichi en calcium, qui contient au moins un composé de lactate-malate de calcium selon l'une quelconque des revendications 8 à 11.

**13.** Produit alimentaire selon la revendication 12,
**caractérisé en ce que**
ce dernier contient une dose de consommation journalière de calcium représentant 15 % à 100 % des besoins quotidiens recommandés.

**14.** Produit alimentaire selon la revendication 12 ou 13,
**caractérisé en ce que**
ce dernier contient en outre au moins un adjuvant.

**15.** Procédé pour la fabrication d'un produit alimentaire enrichi en, au minimum, un composé de lactate-malate de calcium selon l'une quelconque des revendications 12 à 14,

**caractérisé en ce que**
le composé de lactate-malate de calcium est additionné au produit alimentaire et/ou à au moins un produit intermédiaire.

16. Procédé selon la revendication 15,
**caractérisé en ce que**
des parties prédéterminées d'acide lactique et d'acide malique ainsi que des parties d'hydroxyde de calcium, d'oxyde de calcium et/ou de carbonate de calcium sont additionnées au produit alimentaire se présentant sous forme liquide et/ou au produit intermédiaire du produit alimentaire, ce produit intermédiaire se présentant sous forme liquide,

17. Procédé selon la revendication 15 ou 16,
**caractérisé en ce que**
des parties prédéterminées de lactate de calcium et de malate de calcium sont additionnées au produit alimentaire se présentant sous forme liquide et/ou au produit intermédiaire du produit alimentaire, ce produit intermédiaire se présentant sous forme liquide.

18. Procédé selon la revendication 16 ou 17,
**caractérisé en ce que**
l'on chauffe le produit alimentaire se présentant sous forme liquide et/ou le produit intermédiaire se présentant sous forme liquide, lors de l'addition des parties d'acide lactique et/ou d'acide malique et/ou lors de l'addition des parties d'hydroxyde de calcium, d'oxyde de calcium et/ou de carbonate de calcium, à une température à prédéterminer préalablement.

19. Utilisation d'un composé de lactate-malate de calcium selon l'une quelconque des revendications 8 à 11,
**caractérisée en ce que**
le composé de lactate-malate de calcium est additionné à un produit alimentaire.

20. Utilisation d'un composé de lactate-malate de calcium selon l'une quelconque des revendications 8 à 11,
**caractérisée en ce que**
le composé de lactate-malate de calcium est utilisé pour la fabrication d'une préparation pharmaceutique.

21. Utilisation du composé de lactate-malate de calcium selon la revendication 20,
**caractérisée en ce que**
la préparation pharmaceutique est utilisée pour le traitement de l'ostéoporose.

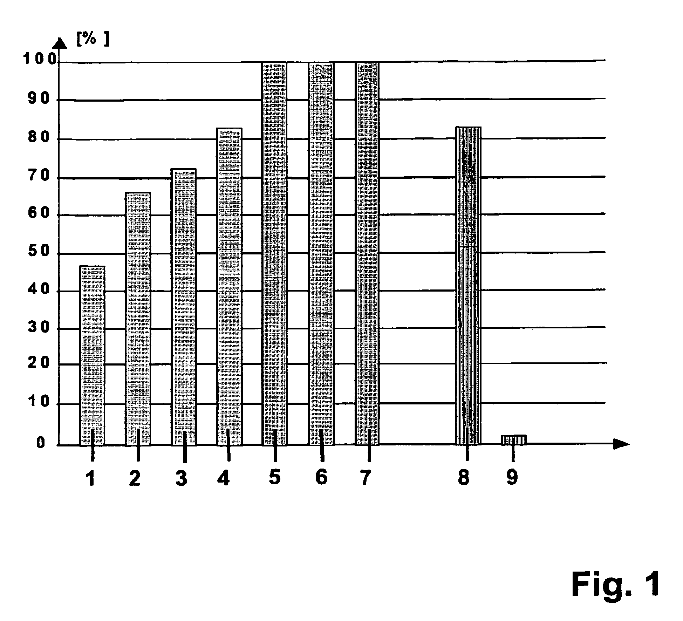

**Fig. 1**

**EP 1 711 072 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 120587
- EP 0301653 A
- JP 9121811 A